# EUROPEAN PATENT APPLICATION

(11) **EP 1 790 717 A2**
(43) Date of publication of application: **30.05.2007**
(21) Application number: 06026756.4
(22) Date of filing: 30.03.1999
(51) Int. Cl.: C12N 9/02, G01N 33/574, C12Q 1/68

(54) **Compositions and methods for the identification of lung tumor cells**

(30) Priority: 31.03.1998 US 80037 P
(62) Divisional of application: 03002096.0
(71) Applicant: Genzyme Corporation, Framingham MA 01701-9322 (US)
(72) Inventor: Beaudry, Gary A., Montclair, NJ 07043 (US); Madden, Stephen L., Sudbury, MA 01776 (US); Bertelsen, Arthur H., Ridgewood, NJ 07450-4715 (US)
(74) Representative: Hermann, Bettina Celia

(57) **Abstract**

This invention provides compositions and methods for the identification of lung cancer cells. In particular, polynucleotide sequences whose presence is indicative of lung cancer are disclosed. In addition, lung cancer can be identified by detecting the presence of the peptides encoded by these sequences. Antibodies to these peptides are also provided. Also included are gene delivery vehicles and host cells comprising these polynucleotides. Kits containing agents and instructions necessary to perform the screening and detecting methods are also provided.

SEQUENCE LISTING

<110> Genzyme Corporation
Beaudry, Gary A.
Madden, Stephen L.
Bertelsen, Arthur H.

<120> COMPOSITIONS AND METHODS FOR THE
IDENTIFICATION OF LUNG TUMOR CELLS

<130> 159792001740

<140> Unassigned
<141> Herewith

<150> 60/080,037
<151> 1998-03-31

<160> 40

<170> FastSEQ for Windows Version 3.0

<210> 1
<211> 10
<212> DNA
<213> Artificial Sequence

<400> 1
aaggagcaag 10

<210> 2
<211> 10
<212> DNA
<213> Artificial Sequence

<400> 2
ctcctgggcg 10

<210> 3
<211> 10
<212> DNA
<213> Artificial Sequence

<400> 3
gatagcacag 10

<210> 4
<211> 10
<212> DNA
<213> Artificial Sequence

<400> 4
tgctgcctgt 10

<210> 5
<211> 10

<212> DNA
<213> Artificial Sequence

<400> 5
ccatttttac                                                              10

<210> 6
<211> 10
<212> DNA
<213> Artificial Sequence

<400> 6
gtccctgcct                                                              10

<210> 7
<211> 10
<212> DNA
<213> Artificial Sequence

<400> 7
caactaattc                                                              10

<210> 8
<211> 10
<212> DNA
<213> Artificial Sequence

<400> 8
gttataagat                                                              10

<210> 9
<211> 10
<212> DNA
<213> Artificial Sequence

<400> 9
tatttttgtt                                                              10

<210> 10
<211> 10
<212> DNA
<213> Artificial Sequence

<400> 10
cagataacat                                                              10

<210> 11
<211> 10
<212> DNA
<213> Artificial Sequence

<400> 11
tgtacctgta                                                              10

<210> 12
<211> 10
<212> DNA
<213> Artificial Sequence

<400> 12
ccaggggaga                                                              10

<210> 13
<211> 10
<212> DNA
<213> Artificial Sequence

<400> 13
gagaaaaccc                                                              10

<210> 14
<211> 10
<212> DNA
<213> Artificial Sequence

<400> 14
atgtacctga                                                              10

<210> 15
<211> 10
<212> DNA
<213> Artificial Sequence

<400> 15
ttctaacata                                                              10

<210> 16
<211> 10
<212> DNA
<213> Artificial Sequence

<400> 16
ggtggtgtct                                                              10

<210> 17
<211> 10
<212> DNA
<213> Artificial Sequence

<400> 17
tactagtcct                                                              10

<210> 18
<211> 10
<212> DNA
<213> Artificial Sequence

<400> 18
atgcagccat                                                              10

<210> 19
<211> 10
<212> DNA
<213> Artificial Sequence

<400> 19

tgctgccctg                                                    10

<210> 20
<211> 10
<212> DNA
<213> Artificial Sequence

<400> 20
tggcccgacg                                                    10

<210> 21
<211> 10
<212> DNA
<213> Artificial Sequence

<400> 21
tgccgttttg                                                    10

<210> 22
<211> 10
<212> DNA
<213> Artificial Sequence

<400> 22
gatgaggaga                                                    10

<210> 23
<211> 10
<212> DNA
<213> Artificial Sequence

<400> 23
tggaaatgac                                                    10

<210> 24
<211> 10
<212> DNA
<213> Artificial Sequence

<400> 24
taatactttt                                                    10

<210> 25
<211> 10
<212> DNA
<213> Artificial Sequence

<400> 25
caataaaatt                                                    10

<210> 26
<211> 10
<212> DNA
<213> Artificial Sequence

<400> 26
aaggctggaa                                                    10

<210> 27
<211> 10
<212> DNA
<213> Artificial Sequence

<400> 27
cggccacaga                                                                10

<210> 28
<211> 10
<212> DNA
<213> Artificial Sequence

<400> 28
gcgcagactt                                                                10

<210> 29
<211> 10
<212> DNA
<213> Artificial Sequence

<400> 29
tatacgctca                                                                10

<210> 30
<211> 10
<212> DNA
<213> Artificial Sequence

<400> 30
tagtaagtca                                                                10

<210> 31
<211> 10
<212> DNA
<213> Artificial Sequence

<400> 31
gcttgaataa                                                                10

<210> 32
<211> 10
<212> DNA
<213> Artificial Sequence

<400> 32
tccccgttac                                                                10

<210> 33
<211> 10
<212> DNA
<213> Artificial Sequence

<400> 33
acctttactg                                                                10

<210> 34
<211> 10

<212> DNA
<213> Artificial Sequence

<400> 34
tccccgtaac                                                              10

<210> 35
<211> 10
<212> DNA
<213> Artificial Sequence

<400> 35
atgatccctg                                                              10

<210> 36
<211> 10
<212> DNA
<213> Artificial Sequence

<400> 36
tatctgtcta                                                              10

<210> 37
<211> 10
<212> DNA
<213> Artificial Sequence

<400> 37
tctgctaaag                                                              10

<210> 38
<211> 10
<212> DNA
<213> Artificial Sequence

<400> 38
tccctaatta                                                              10

<210> 39
<211> 10
<212> DNA
<213> Artificial Sequence

<400> 39
gaatctggag                                                              10

<210> 40
<211> 10
<212> DNA
<213> Artificial Sequence

<400> 40
gacgactgac                                                              10

## Description

### CROSS-REFERENCE TO RELATED APPLICATION

This application claims priority under 35 U.S.C. § 119(e) to U.S. Provisional Application no. 60/080,037, filed March 30, 1999, the contents of which are hereby incorporated by reference into the present disclosure.

### TECHNICAL FIELD

This invention relates to the isolation and characterization of novel transcripts expressed in lung tumor or cancer cells.

### BACKGROUND

Despite numerous advances in medical research, cancer remains the second leading cause of death in the United States. In the industrialized nations, roughly one in five persons will die of cancer. Traditional modes of clinical care, such as surgical resection, radiotherapy and chemotherapy, have a significant failure rate, especially for solid tumors. Failure occurs either because the initial tumor is unresponsive, or because of recurrence due to regrowth at the original site and/or metastases.

Lung cancer is one of the most common malignancies worldwide and is the leading cause of cancer death in man. See, American Cancer Society, Cancer facts and figures, 1996, Atlanta. Approximately 178,100 new cases of lung cancer were to be diagnosed in 1997, accounting for 13% of cancer diagnoses. An estimated 160,400 deaths due to lung cancer would occur in 1997 accounting for 29% of all cancer deaths. The one-year survival rates for lung cancer have increased from 32% in 1973 to 41% in 1993, largely due to improvements in surgical techniques. The 5 year survival rate for all stages combined is only 14%. The survival rate is 48% for cases detected when the disease is still localized, but only 15% of lung cancers are discovered that early. Among various forms of lung cancer, non-small cell lung cancer (NSCLC) accounts for nearly 80% of all new lung cancer cases each year. For patients diagnosed with NSCLC, surgical resection offers the only chance of meaningful survival. On the other hand, small cell lung cancer is the most malignant and fastest growing form of lung cancer and accounts for the rest of approximately 20% of new cases of lung cancer. The primary tumor is generally responsive to chemotherapy, but is followed by widespread metastasis. The median survival time at diagnosis is approximately 1 year, with a 5 year survival rate of 5%.

In spite of major advances in cancer therapy including improvements in surgical resection, radiation treatment and chemotherapy, successful intervention for lung cancer in particular, relies on early detection of the cancerous cells. Neoplasia resulting in benign tumors may be completely cured by removing the mass surgically. If a tumor becomes malignant, as manifested by invasion of surrounding tissue, it becomes much more difficult to eradicate. Therefore, there remains a considerable need in the art for the development of methods for detecting the disease at the early stage. There also exits a pressing need in the art for developing diagnostic method to monitor or progenies the progression of the disease as well as methods to treat various conditions. However, the vast variability in the nature of the disease has rendered the search for cellular markers, such as genes that are preferably overexposed in primary lung cancer cells and useful for diagnostic and therapeutic methods, difficult.

This invention provides compositions and methods that are useful for the early diagnosis of a lung neoplasm.

### DISCLOSURE OF THE INVENTION

The present invention provides methods for aiding in the diagnoses of the neoplastic condition of a lung cell, and methods for screening for a potential therapeutic agent for the reversal of the neoplastic condition. It also provides genes that are overexpressed in lung cancer cells, as well as novel genes that are expressed in primary lung cancer cells.

Accordingly, one embodiment of this invention is a method of diagnosing the neoplastic condition of a lung cell by screening for the presence of a transcript which is expressed in a neoplastic lung cell but not normal lung cells or tissue. In one aspect of this invention, the transcript is from a known gene, which until the subject invention, was unknown to be differentially expressed in lung cancer or lung tumor tissue, and not expressed in normal lung tissue, or expressed to a lesser extent than in lung cancer tissue or cells. In a separate embodiment, only a fragment of the transcript is known as a prior Expressed Sequence Tag ("EST"). However, the presence, absence or differential expression of the EST in lung cancer tissue versus normal lung tissue remained unknown until Applicants' invention. The corresponding full length open reading frame of the EST and corresponding genomic DNA may or may not be known to those of skill in the art.

In a further embodiment, the polynucleotide identifies a previously unidentified or uncharacterized fragment or full length coding region (open reading frame) of a gene or polynucleotide. This invention also provides the method to identify the gene, fragment or full length coding region corresponding to the isolated polynucleotide of this invention. The genes, fragments, and full length coding regions obtainable by these methods are further claimed herein.

Another embodiment of the invention is a screen for a potential therapeutic agent for the reversal of the neoplastic condition of a lung cell, wherein the cell is characterized by the presence of at least one of a polynucleotide identified herein by SEQ ID NOS. 1 through 40. The method comprises contacting a neoplastic lung cell or tissue with an effective amount of a potential agent and assaying for reversal of the neoplastic condition. In another embodiment, the polynucleotide used in the method is obtained by identification of larger fragment or full-length coding sequence corresponding to the sequence of SEQ ID NOS: 1-40.

In another embodiment, the invention includes a method of diagnosing the condition of a lung cell to determine whether it is predisposed to or is in a neoplastic condition. The method requires screening for the presence of a peptide transcribed from a polynucleotide which is expressed in a neoplastic lung cell but not normal lung cells or tissue. In one aspect of this invention, the peptide is from a known gene, which until the subject invention, was unknown to be differentially expressed in lung cancer or lung tumor tissue, and not expressed in normal lung tissue. In a separate embodiment, only a fragment of the polynucleotide encoding the peptide is known as a prior Expressed Sequence Tag ("EST"). The full length open reading frame of the gene corresponding to the EST may or may not be characterized. However, the presence or absence of the EST gene product in lung cancer tissue versus normal lung tissue previously had not been characterized. The novel peptides are also included within this invention.

The invention also includes kits for use in the detection and screening methods described herein. The kits contain, in a suitable packaging, the agents and reagents necessary to practice the claimed invention as well as instructions to conduct the screen.

Also described are non-human transgenic animals that are genetically modified so that a polynucleotide sequence associated with lung cancer has been disrupted. The disrupted polynucleotide sequence may be those shown in SEQ ID NOS: 1-40, a polynucleotide corresponding to these sequences, or sequences obtained by identification of larger fragment or full-length coding sequence corresponding to SEQ ID NOS: 1-40.

### MODE(S) FOR CARRYING OUT THE INVENTION

Throughout this disclosure, various publications, patents and published patent specifications are referenced by an identifying citation. The disclosures of these publications, patents and published patent specifications are hereby incorporated by reference into the present disclosure to more fully describe the state of the art to which this invention pertains.

### Definitions

The practice of the present invention will employ, unless otherwise indicated, conventional techniques of immunology, molecular biology, microbiology, cell biology and recombinant DNA, which are within the skill of the art. See, *e.g.,* Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney, ed. (1987)).

As used in the specification and claims, the singular form "a", "an" and "the" include plural references unless the context clearly dictates otherwise. For example, the term "a cell" includes a plurality of cells, including mixtures thereof.

As used herein, the term "comprising" is intended to mean that the compositions and methods include the recited elements, but not excluding others. "Consisting essentially of" when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of" shall mean excluding more than trace elements of other ingredients and substantial method steps for administering the compositions of this invention. Embodiments defined by each of these transition terms are within the scope of this invention.

The term "isolated" means separated from constituents, cellular and otherwise, in which the polynucleotide, peptide, polypeptide, protein, antibody, or fragments thereof, are normally associated with in nature. In one aspect of this invention, an isolated polynucleotide is separated from the 3' and 5' contiguous nucleotides with which it is normally associated with in its native or natural environment, e.g., on the chromosome. As is apparent to those of skill in the art, a non-naturally occurring polynucleotide, peptide, polypeptide, protein, antibody, or fragments thereof, does not require "isolation" to distinguish it from its naturally occurring counterpart. In addition, a "concentrated", "separated" or "diluted" polynucleotide, peptide, polypeptide, protein, antibody, or fragments thereof, is distinguishable from its naturally occurring counterpart in that the concentration
or number of molecules per volume is greater than "concentrated" or less than "separated" than that of its naturally occurring counterpart. A polynucleotide, peptide, polypeptide, protein, antibody, or fragments thereof, which differs from the naturally occurring counterpart in its primary sequence or for example, by its glycosylation pattern, need not be present in its isolated form since it is distinguishable from its naturally occurring counterpart by its primary sequence, or alternatively, by another characteristic such as glycosylation pattern. Although not explicitly stated for each of the inventions disclosed herein, it is to be understood that all of the above embodiments for each of the compositions disclosed below and under the appropriate conditions, are provided by this invention. Thus, a non-naturally occurring polynucleotide is provided as a separate embodiment from the isolated naturally occurring polynucleotide. A protein produced in a bacterial cell is provided as a separate embodiment from the naturally occurring protein isolated from a eucaryotic cell in which it is produced in nature.

The terms "polynucleotide" and "oligonucleotide" are used interchangeably, and refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: a gene or gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component. The term also refers to both double-and single-stranded molecules. Unless otherwise specified or required, any embodiment of this invention that is a polynucleotide encompasses both the double-stranded form and each of two complementary single-stranded forms known or predicted to make up the double-stranded form.

A polynucleotide is composed of a specific sequence of four nucleotide bases: adenine (A); cytosine (C); guanine (G); thymine (T); and uracil (U) for guanine when the polynucleotide is RNA. Thus, the term "polynucleotide sequence" is the alphabetical representation of a polynucleotide molecule. This alphabetical representation can be input into databases in a computer having a central processing unit and used for bioinformatics applications such as functional genomics and homology searching.

A "gene" refers to a polynucleotide containing at least one open reading frame that is capable of encoding a particular polypeptide or protein after being transcribed and translated. Any of the polynucleotides sequences described herein may be used to identify larger fragments or full-length coding sequences of the gene with which they are associated. Methods of isolating larger fragment sequences are known to those of skill in the art, some of which are described herein. A "oncogene" refers to a polynucletide containing at least one open reading frame, that is capable of transforming a normal cell to a cancerous (or neoplastic or tumor) cell when introduced into a host cell. Oncogenes are often altered forms of the cellular counterpart, namely the "proto-oncogenes" that are incapable of cell transformation when expressed at the level present in a non-cancer cell.

A "gene product" refers to the amino acid (e.g., peptide or polypeptide) generated when a gene is transcribed and translated.

As used herein a second polynucleotide "corresponds to" another (a first) polynucleotide if it is related to the first polynucleotide by any of the following relationships:
1) The second polynucleotide comprises the first polynucleotide and the second polynucleotide encodes a gene product.
2) The second polynucleotide is 5' or 3' to the first polynucleotide in cDNA, RNA, genomic DNA, or fragment of any of these polynucleotides. For example, a second polynucleotide may be a fragment of a gene that includes the first and second polynucleotides. The first and second polynucleotides are related in that they are components of the gene coding for a gene product, such as a protein or antibody. However, it is not necessary that the second polynucleotide comprises or overlaps with the first polynucleotide to be encompassed within the definition of "corresponding to" as used herein. For example, the first polynucleotide may be a fragment of a 3' untranslated region of the second polynucleotide. The first and second polynucleotide may be fragment of a gene coding for a gene product. The second polynucleotide may be an exon of the gene while the first polynucleotide may be an intron of the gene.
3) The second polynucleotide is the complement of the first polynucleotide.

A "probe" when used in the context of polynucleotide manipulation refers to an oligonucleotide that is provided as a reagent to detect a target potentially present in a sample of interest by hybridizing with the target. Usually, a probe will comprise a label or a means by which a label can be attached, either before or subsequent to the hybridization reaction. Suitable labels include, but are not limited to radioisotopes, fluorochromes, chemiluminescent compounds, dyes, and proteins, including enzymes.

A "primer" is a short polynucleotide, generally with a free 3' -OH group that binds to a target or "template" potentially present in a sample of interest by hybridizing with the target, and thereafter promoting polymerization of a polynucleotide complementary to the target. A "polymerase chain reaction" ("PCR") is a reaction in which replicate copies are made of a target polynucleotide using a "pair of primers" or a "set of primers" consisting of an "upstream" and a "downstream" primer, and a catalyst of polymerization, such as a DNA polymerase, and typically a thermally-stable polymerase enzyme. Methods for PCR are well known in the art, and taught, for example in "PCR: A PRACTICAL APPROACH" (M. MacPherson et al., IRL Press at Oxford University Press (1991)). All processes of producing replicate copies of a polynucleotide, such as PCR or gene cloning, are collectively referred to herein as "replication." A primer can also be used as a probe in hybridization reactions, such as Southern or Northern blot analyses. Sambrook et al., *supra.*

A "sequence tag" or "SAGE tag" is a short sequence, generally under about 20 nucleotides, that occurs in a certain position in messenger RNA. The tag can be used to identify the corresponding transcript and gene from which it was transcribed. A "ditag" is a dimer of two sequence tags.

The term "cDNAs" refers to complementary DNA, that is mRNA molecules present in a cell or organism made in to cDNA with an enzyme such as reverse transcriptase. A "cDNA library" is a collection of all of the mRNA molecules present in a cell or organism, all turned into cDNA molecules with the enzyme reverse transcriptase, then inserted into "vectors" (other DNA molecules that can continue to replicate after addition of foreign DNA). Exemplary vectors for libraries include bacteriophage (also known as "phage"), viruses that infect bacteria, for example, lambda phage. The library can then be probed for the specific cDNA (and thus mRNA) of interest.

A "gene delivery vehicle" is defined as any molecule that can carry inserted one or more polynucleotides into a host cell. Examples of gene delivery vehicles are liposomes, biocompatible polymers, including natural polymers and synthetic polymers; lipoproteins; polypeptides; polysaccharides; lipopolysaccharides; artificial viral envelopes; metal particles; and bacteria, viruses and viral vectors, such as baculovirus, adenovirus and retrovirus, bacteriophage, cosmid, plasmid, fungal vector and other recombination vehicles typically used in the art which have been described for replication and/or expression in a variety of eucaryotic and procaryotic hosts. The gene delivery vehicles may be used for replication of the inserted polynucleotide, gene therapy as well as for simple polypeptide and protein expression.

"Vector" means a self-replicating nucleic acid molecule that transfers an inserted polynucleotide into and/or between host cells. The term is intended to include vectors that function primarily for insertion of a nucleic acid molecule into a cell, replication vectors that function primarily for the replication of nucleic acid and expression vectors that function for transcription and/or translation of the DNA or RNA. Also intended are vectors that provide more than one of the above functions.

"Host cell" is intended to include any individual cell or cell culture which can be or has been a recipient for vectors or for the incorporation of exogenous nucleic acid molecules, polynucleotides and/or proteins. It also is intended to include progeny of a single cell. The progeny may not necessarily be completely identical (in morphology or in genomic or total DNA complement) to the original parent cell due to natural, accidental, or deliberate mutation. The cells may be procaryotic or eucaryotic, and include but are not limited to bacterial cells, yeast cells, insect cells, animal cells, and mammalian cells, e.g., murine, rat, simian or human.

The term "genetically modified" means containing and/or expressing a foreign gene or nucleic acid sequence which in turn, modifies the genotype or phenotype of the cell or its progeny. In other words, it refers to any addition, deletion or disruption to a cell's endogenous nucleotides.

As used herein, "expression" refers to the process by which polynucleotides are transcribed into mRNA and translated into peptides, polypeptides, or proteins. If the polynucleotide is derived from genomic DNA, expression may include splicing of the mRNA, if an appropriate eucaryotic host is selected. Regulatory elements required for expression include promoter sequences to bind RNA polymerase and transcription initiation sequences for ribosome binding. For example, a bacterial expression vector includes a promoter such as the *lac* promoter and for transcription initiation the Shine-Dalgarno sequence and the start codon AUG (Sambrook et al. (1989) *supra*). Similarly, an eucaryotic expression vector includes a heterologous or homologous promoter for RNA polymerase II, a downstream polyadenylation signal., the start codon AUG, and a termination codon for detachment of the ribosome. Such vectors can be obtained commercially or assembled by the sequences described in methods well known in the art, for example, the methods described below for constructing vectors in general.

"Differentially expressed" as applied to a gene, refers to the differential production of the mRNA transcribed from the gene or the protein product encoded by the gene. A differentially expressed gene may be overexpressed or underexpressed as compared to the expression level of a normal or control cell. In one aspect, it refers to a differential that is 2.5 times, preferably 5 times, or preferably 10 times higher or lower than the expression level detected in a control sample. The term "differentially expressed" also refers to nucleotide sequences in a cell or tissue which are expressed where silent in a control cell or not expressed where expressed in a control cell.

The term "polypeptide" is used in its broadest sense to refer to a compound of two or more subunit amino acids, amino acid analogs, or peptidomimetics. The subunits may be linked by peptide bonds. In another embodiment, the subunit may be linked by other bonds, e.g. ester, ether, etc. As used herein the term "amino acid" refers to either natural and/or unnatural or synthetic amino acids, including glycine and both the D or L optical isomers, and amino acid analogs and peptidomimetics. A peptide of three or more amino acids is commonly called an oligopeptide if the peptide chain is short. If the peptide chain is long, the peptide is commonly called a polypeptide or a protein.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PCR reaction, or the enzymatic cleavage of a polynucleotide by a ribozyme.

Hybridization reactions can be performed under conditions of different "stringency". In general, a low stringency hybridization reaction is carried out at about 40 °C in 10 X SSC or a solution of equivalent ionic strength/temperature. A moderate stringency hybridization is typically performed at about 50 °C in 6 X SSC, and a high stringency hybridization reaction is generally performed at about 60 °C in 1X SSC.

When hybridization occurs in an antiparallel configuration between two single-stranded polynucleotides, the reaction is called "annealing" and those polynucleotides are described as "complementary". A double-stranded polynucleotide can be "complementary" or "homologous" to another polynucleotide, if hybridization can occur between one of the strands of the first polynucleotide and the second. "Complementarity" or "homology" (the degree that one polynucleotide is complementary with another) is quantifiable in terms of the proportion of bases in opposing strands that are expected to form hydrogen bonding with each other, according to generally accepted base-pairing rules.

A polynucleotide or polynucleotide region (or a polypeptide or polypeptide region) has a certain percentage (for example, 80%, 85%, 90%, or 95%) of "sequence identity" to another sequence means that, when aligned, that percentage of bases (or amino acids) are the same in comparing the two sequences. This alignment and the percent homology or sequence identity can be determined using software programs known in the art, for example those described in CURRENT PROTOCOLS IN MOLECULAR BIOLOGY (F.M. Ausubel et al., eds., 1987) Supplement 30, section 7.7.18, Table 7.7.1. Preferably, default parameters are used for alignment. A preferred alignment program is BLAST, using default parameters. In particular, preferred programs are BLASTN and BLASTP, using the following default parameters: Genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Details of these programs can be found at the following Internet address: http://www.ncbi.rdm.nih.gov/cgi-bin/BLAST.

As used herein, "solid phase support" or "solid support", used interchangeably, is not limited to a specific type of support. Rather a large number of supports are available and are known to one of ordinary skill in the art. Solid phase supports include silica gels, resins, derivatized plastic films, glass beads, cotton, plastic beads, alumina gels. As used herein, "solid support" also includes synthetic antigen-presenting matrices, cells, and liposomes. A suitable solid phase support may be selected on the basis of desired end use and suitability for various protocols. For example, for peptide synthesis, solid phase support may refer to resins such as polystyrene (*e.g.*, PAM-resin obtained from Bachem Inc., Peninsula Laboratories, etc.), POLYHIPE® resin (obtained from Aminotech, Canada), polyamide resin (obtained from Peninsula Laboratories), polystyrene resin grafted with polyethylene glycol (TentaGel®, Rapp Polymere, Tubingen, Germany) or polydimethylacrylamide resin (obtained from Milligen/Biosearch, California).

A polynucleotide of the invention also can be attached to a solid support for use in high throughput screening assays. PCT WO 97/10365, for example, discloses the construction of high density oligonucleotide chips. See also, U.S. Patent Nos. 5,405,783; 5,412,087; and 5,445,934. Using this method, the probes are synthesized on a derivatized glass surface. Photoprotected nucleoside phosphoramidites are coupled to the glass surface, selectively deprotected by photolysis through a photolithographic mask, and reacted with a second protected nucleoside phosphoramidite. The coupling/deprotection process is repeated until the desired probe is complete.

An "antibody" is an immunoglobulin molecule capable of binding an epitope present on an antigen. As used herein, the term encompasses not only intact immunoglobulin molecules such as monoclonal and polyclonal antibodies, but also anti-idiotypic antibodies, mutants, fragments, fusion proteins, bi-specific antibodies, humanized proteins and modifications of the immunoglobulin molecule that comprise an antigen recognition site of the required specificity.

An "antibody complex" is the combination of antibody (as defined above) and its binding partner or ligand.

The term "recognized" intends that an antibody of the present invention binds with either or both of higher affinity or avidity to an epitope present on a polypeptide of this invention than for an unrelated polypeptide. Assays for avidity and affinity of an antibody complex are known in the art.

As used herein, the terms "neoplastic cells", "neoplasia", "tumor", "tumor cells", "cancer" and "cancer cells", (used interchangeably) refer to cells which exhibit relatively autonomous growth, so that they exhibit an aberrant growth phenotype characterized by a significant loss of control of cell proliferation (i.e., de-regulated cell division). Neoplastic cells can be malignant or benign. A metastatic cell or tissue means that the cell can invade and destroy neighboring body structures.

"Suppressing" tumor growth indicates a growth state that is curtailed when compared to growth without contact with educated, antigen-specific immune effector cells described herein. Tumor cell growth can be assessed by any means known in the art, including, but not limited to, measuring tumor size, determining whether tumor cells are proliferating using a ³H-thyinidine incorporation assay, or counting tumor cells. "Suppressing" tumor cell growth means any or all of the following states: slowing, delaying, and stopping tumor growth, as well as tumor shrinkage.

Hyperplasia is a form of controlled cell proliferation involving an increase in cell number in a tissue or organ, without significant alteration in structure or function. Metaplasia is a form of controlled cell growth in which one type of fully differentiated cell substitutes for another type of differentiated cell. Metaplasia can occur in epithelial or connective tissue cells. Atypical metaplasia involves a somewhat disorderly metaplastic epithelium.

As used herein, the term "reversing the neoplastic state of the cell" is intended to include apoptosis, necrosis or any other means of preventing cell division, reduced tumorigenicity, loss of pharmaceutical resistance, maturation, differentiation or reversion of the neoplastic phenotypes as described herein.

A "composition" is intended to mean a combination of active agent and another compound or composition, inert (for example, a detectable agent or label) or active, such as an adjuvant.

A "pharmaceutical composition" is intended to include the combination of an active agent with a carrier, inert or active, making the composition suitable for diagnostic or therapeutic use *in vitro, in vivo* or *ex vivo.*

As used herein, the term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives. For examples of carriers, stabilizers and adjuvants, see Martin, REMINGTON'S PHARM. SCI., 15th Ed. (Mack Publ. Co., Easton (1975)).

An "effective amount" is an amount sufficient to effect beneficial or desired results. An effective amount can be administered in one or more administrations, applications or dosages.

A "subject," "individual" or "patient" is used interchangeably herein, which refers to a vertebrate, preferably a mammal, more preferably a human. Mammals include, but are not limited to, murines, simians, humans, farm animals, sport animals, and pets.

A "control" is an alternative subject or sample used in an experiment for comparison purpose. A control can be "positive" or "negative". For example, where the purpose of the experiment is to determine a correlation of an altered expression level of a proto-oncogene with a particular type of cancer, it is generally preferable to use a positive control (a subject or a sample from a subject, carrying such alteration and exhibiting syndromes characteristic of that disease), and a negative control (a subject or a sample from a subject lacking the altered expression and clinical syndrome of that disease).

A "transgenic animal" refers to a genetically engineered animal or offspring of genetically engineered animals. The transgenic animal may contain genetic material from at least one unrelated organism (such as from a bacteria, virus, plant, or other animal) or may contain a mutation which interferes with expression of a gene product.

A systematic analysis of transcripts present in non-small cell lung cancer (NSCLC) was performed by Serial Analysis of Gene Expression ("SAGE") (U.S. Patent No. 5,695,937). SAGE analysis involves identifying nucleotide sequences expressed in cells. Briefly, SAGE analysis began with providing complementary deoxyribonucleic acid (cDNA) from (1) the neoplastic population and (2) normal cells. Both cDNAs were linked to primer sites. Sequence tags were then created, for example, using the appropriate primers to amplify the DNA. By measuring the differences in these tags between the two cell types, sequences that are aberrantly expressed in the neoplastic cell population were identified.

### Polynucleotides and Claimed Utilities

Using SAGE, the sequence tags represented by SEQ ID NOS. I through 40 were identified and further characterized. Thus, this invention provides a population of polynucleotides represented by SEQ ID NOS. 1 through 40, or their respective complements. Compositions (described in detail below) and a database (also described below) containing these polynucleotides also are provided by this invention.

Polynucleotides corresponding to SEQ ID NOS. 24 to 26, 29, 32 to 35, and 38, represent previously unidentified polynucleotides or genes. Thus, this invention also provides a population of polynucleotides comprising at least one polynucleotide having a sequence selected from the group consisting of SEQ ID NOS. 24 to 26, 29, 32 to 35, and 38, or their respective complements. Also provided by this invention are polynucleotides that correspond to polynucleotides having a sequence of SEQ ID NOS. 24 to 26, 29, 32 to 35, and 38. In one embodiment, these polynucleotides are obtained by identification of a larger fragment or full-length coding sequence of these polynucleotides. Gene delivery vehicles, host cells, compositions (described in detail below) and databases (also described below) containing these polynucleotides also are provided by this invention.

The invention also encompasses polynucleotides which differ from that of the polynucleotides described above, but which produce the same phenotypic effect, such as the allele. These altered, but phenotypically equivalent polynucleotides are referred to "equivalent nucleic acids." This invention also encompasses polynucleotides characterized by changes in non-coding regions that do not alter the phenotype of the polypeptide produced therefrom when compared to the polynucleotide herein. This invention further encompasses polynucleotides, which hybridize to the polynucleotides of the subject invention under conditions of moderate or high stringency. Alternatively, the polynucleotides are at least 85%, or at least 90%, or more preferably, greater or equal to 95% identical as determined by a sequence alignment program when run under default parameters.

Also provided in the present invention are polypeptides encoded by an EST or by a known gene, which until the instant invention, was unknown to be differentially expressed in lung cancer. Further embodied in the polypeptides of the present invention are novel sequences including fragments thereof or complements thereof that hybridize to the same coding sequence, to which the polypeptide encoded by the nucleotides depicted in SEQ ID NOS. 24 to 26, 29, 32 to 35 or 38 hybridizes. These sequences are unique in their over-representation in lung cancer cells and not in normal lung cells, thus being particularly useful in detecting a lung cancer cell.

The process of identification of larger fragment or the full-length coding sequence to which the partial sequence depicted in SEQ ID NOS. 24-26, 29, 32-35 or 38 hybridizes preferably involves the use of the methods and reagents provided in this invention, either singularly or in combination. Any conventional recombinant DNA techniques applicable for isolating polynucleotides can also be used.

One such method involves the 5'-RACE-PCR technique, in which the poly-A mRNA that contains the coding sequence of particular interest is first reverse transcribed with a 3'-primer comprising the sequence disclosed herein. The newly synthesized cDNA strand is then tagged with an anchor primer with a known sequence, which preferably contains a convenient cloning restriction site attached at the 5'end. The tagged cDNA is then amplified with the 3'-primer (or a nested primer sharing sequence homology to the internal sequences of the coding region) and the 5'-anchor primer. The amplification may be conducted under conditions of various levels of stringency to optimize the amplification specificity. 5'-RACE-PCR can be readily performed using commercial kits (available from, e.g., BRL Life Technologies Inc, Clotech) according to the manufacturer's instructions.

Isolating the complete coding sequence of a gene can also be carried out in a hybridization assay using a suitable probe. The probe preferably comprises at least 10 nucleotides, and more preferably exhibits sequence homology to the polynucleotide depicted in SEQ ID NOS. 24-26,29, 32-35 or 38. Other high throughput screens for cDNAs, such as those involving gene chip technology, can also be employed in obtaining the complete cDNA sequence.

In addition, databases exist that reduce the complexity ofESTs by assembling contiguous EST sequences into tentative genes. For example, TIGR has assembled human ESTs into a datable called THC for tentative human consensus sequences. The THC database allows for a more definitive assignment compared to ESTs alone. Software programs exist (TIGR assembler and TIGEM EST assembly machine and contig assembly program (see Huang, X. (1996) Genomics 33 :21-23)) that allow for assembling ESTs into contiguous sequences from any organism.

Alternatively, mRNA from a sample preparation is used to construct cDNA library in the ZAP Express vector following the procedure described in Velculescu et al. (1997) Science 270:484. The ZAP Express cDNA synthesis kit (Stratagene) is used accordingly to the manufacturer's protocol. Plates containing 250 to 2000 plaques are hybridized as described in Rupert et al. (1988) Mol. Cell. Bio. 8:3104 to oligonucleotide probes with the same conditions previously described for standard probes except that the hybridization temperature is reduced to room temperature. Washes are performed in 6X standard-saline-citrate 0.1 % SDS for 30 minutes at room temperature. The probes are labeled with ³²P-ATP through use of T4 polynucleotide kinase.

A partial cDNA (3' fragment) can be isolated by 3' directed PCR reaction. This procedure is a modification of the protocol described in Polyak et al. (1997) Nature 389:300. Briefly, the procedure uses SAGE tags in PCR reaction such that the resultant PCR product contains the SAGE tag of interest as well as additional cDNA, the length of which is defined by the position of the tag with respect to the 3' end of the cDNA. The cDNA product derived from such a transcript driven PCR reaction can be used for many applications.

RNA from a source believed to express the cDNA corresponding to a given tag is first converted to double-stranded cDNA using any standard cDNA protocol. Similar conditions used to generate cDNA for SAGE library construction can be employed except that a modified oligo-dT primer is used to derive the first strand synthesis. For example, the oligonucleotide of composition 5'-**B**-TCC GGC GCG CCG TTT T CC CAG TCA CGA(30)-3', contains a poly-T stretch at the 3' end for hybridization and priming from poly-A tails, an M13 priming site for use in subsequent PCR steps, a 5' Biotin label (**B**) for capture to strepavidin-coated magnetic beads, and an AscI restriction endonuclease site for releasing the cDNA from the streptavidin-coated magnetic beads. Theoretically, any sufficiently-sized DNA region capable of hybridizing to a PCR primer can be used as well as any other 8 base pair recognizing endonuclease.

cDNA constructed utilizing this or similar modified oligo-dT primer is then processed exactly as described in U.S. Patent No. 5,695,937 up until adapter ligation where only one adapter is ligated to the cDNA pool. After adapter ligation, the cDNA is released from the streptavidin-coated magnetic beads and is then used as a template for cDNA amplification.

Various PCR protocols can be employed using PCR priming sites within the 3' modified oligo-dT primer and the SAGE tag. The SAGE tag-derived PCR primer employed can be of varying length dictated by 5' extension of the tag into the adaptor sequence. cDNA products are now available for a variety of applications.

This technique can be further modified by: (1) altering the length and/or content of the modified oligo-dT primer; (2) ligating adaptors other than that previously employed within the SAGE protocol; (3) performing PCR from template retained on the streptavidin-coated magnetic beads; and (4) priming first strand cDNA synthesis with non-oligo-dT based primers.

Gene trapper technology can also be used. The reagents and manufacturer's instructions for this technology are commercially available from Life Technologies, Inc., Gaithersburg, Maryland. Briefly, a complex population of single-stranded phagemid DNA containing directional cDNA inserts is enriched for the target sequence by hybridization in solution to a biotinylated oligonucleotide probe complementary to the target sequence. The hybrids are captured on streptavidin-coated paramagnetic beads. A magnet retrieves the paramagnetic beads from the solution, leaving nonhybridized single-stranded DNAs behind. Subsequently, the captured single-stranded DNA target is released from the biotinylated oligonucleotide. After release, the cDNA clone is further enriched by using a nonbiotinylated target oligonucleotide to specifically prime conversion of the single-stranded target to double-stranded DNA. Following transformation and plating, typically 20% to 100% of the colonies represent the cDNA clone of interest. To identify the desired cDNA clone, the colonies may be screened by colony hybridization using the ³²P-labeled oligonucleotide as described above for solution hybridization, or alternatively by DNA sequencing and alignment of all sequences obtained from numerous clones to determine a consensus sequence.

The polynucleotides of this invention can be replicated using PCR. PCR technology is the subject matter of United States Patent Nos. 4,683,195, 4,800,159, 4,754,065, and 4,683,202 and described in PCR: THE POLYMERASE CHAIN REACTION (Mullis et al. eds, Birkhauser Press, Boston (1994)) and references cited therein.

Alternatively, one of skill in the art can use the sequences provided herein and a commercial DNA synthesizer to replicate the DNA. Accordingly, this invention also provides a process for obtaining the polynucleotides of this invention by providing the linear sequence of the polynucleotide, appropriate primer molecules, chemicals such as enzymes and instructions for their replication and chemically replicating or linking the nucleotides in the proper orientation to obtain the polynucleotides. In a separate embodiment, these polynucleotides are further isolated. Still further, one of skill in the art can insert the polynucleotide into a suitable replication vector and insert the vector into a suitable host cell (procaryotic or eucaryotic) for replication and amplification. The DNA so amplified can be isolated from the cell by methods well known to those of skill in the art. A process for obtaining polynucleotides by this method is further provided herein as well as the polynucleotides so obtained.

RNA can be obtained by first inserting a DNA polynucleotide into a suitable host cell. The DNA can be inserted by any appropriate method, e.g., by the use of an appropriate gene delivery vehicle (e.g., liposome, plasmid or vector) or by electroporation. When the cell replicates and the DNA is transcribed into RNA; the RNA can then be isolated using methods well known to those of skill in the art, for example, as set forth in Sambrook et al. (1989) *Supra.* For instance, mRNA can be isolated using various lytic enzymes or chemical solutions according to the procedures set forth in Sambrook et al. (1989) *Supra* or extracted by nucleic-acid-binding resins following the accompanying instructions provided by manufactures.

As noted above, this invention further provides various methods for aiding in the diagnosis of the neoplastic state of a lung cell that is or is predisposed to be characterized by abnormal cell growth in the form of, e.g., malignancy, hyperplasia or metaplasia. The neoplastic state of a cell generally is determined by noting whether the growth of the cell is not governed by the usual limitation of normal growth. For the purposes of this invention, the term also is to include genotypic changes that occur prior to detection of this growth in the form of a tumor and that are causative of these phenotypic changes. The phenotypic changes associated with the neoplastic state of a cell (a set of *in vitro* characteristics associated with a tumorigenic ability *in vivo*) include a more rounded cell morphology, looser substratum attachment, loss of contact inhibition, loss of anchorage dependence, release of proteases such as plasminogen activator, increased sugar transport, decreased serum requirement, expression of fetal antigens and etc. (See Luria, et al. (1978) GENERAL VIROLOGY 3d Ed., 436-446, (John Wiley & Sons, New York)).

The methods of this invention screen for the presence of polynucleotides which can be identified by at least one of the sequences provided in SEQ ID NOS. 1 through 40. Also provided are the polynucleotides of SEQ ID NOS 1 through 40 and the gene products of these polynucleotides. In a separate embodiment, the presence of a polypeptide or protein which is transcribed and translated (or is a subfragment of the gene product corresponding to the polynucleotide transcript) is indicative of the presence of the neoplastic condition of the cell. The transcript is identified by screening for mRNA that hybridizes to a probe comprising a polynucleotide of any of SEQ ID NOS. 1 through 40, or their complements, or by amplifying nucleic acid using a primer comprising a polynucleotide of any of SEQ ID NOS. 1 through 40, or their complement. PCR is the preferred method of amplifying sequences, although traditional cloning techniques also will amplify a known sequence and therefore fall within the scope of this invention. Sequences of polynucleotides isolated from samples suspected of containing lung cancer cells can be compared against a database that comprises SEQ. ID NOS. 1 through 40 using an algorithm that identified sequence homologies. The presence of high sequence identity between the sequence sample and at least one of the sequences in the database is a positive indication that the sample contains a lung cancer cell.

These methods can be used for aiding in the diagnosis of a lung cancer such as squamous cell lung cancer by detecting a genotype that is correlated with a phenotype characteristic of primary lung tumor cells. Thus, by detecting this genotype prior to tumor growth, one can predict a predisposition to cancer or provide early diagnosis.

Cell or tissue samples used for this invention encompass body fluid, solid tissue samples, tissue cultures or cells derived therefrom and the progeny thereof, and sections or smears prepared from any of these sources, or any other samples that may contain a lung cell. Thus, a preferred sample is one that is prepared from a subject's lung tissue.

In assaying for mRNA that hybridizes to the transcript, mRNA contained in the aforementioned samples is first extracted according to standard methods in the art. For instance, in RNA can be isolated using various lytic enzymes or chemical solutions according to the procedures set forth in Sambrook et al. (1989), *supra* or extracted by nucleic-acid-binding resins following the accompanying instructions provided by manufactures. The mRNA contained in the extracted nucleic acid sample is then detected by hybridization (e.g. Northern blot analysis) and/or amplification procedures according to methods widely known in the art or based on the methods exemplified herein.

Nucleic acid molecules having at least 10 nucleotides and exhibiting sequence complementarity or homology to SEQ ID NOS. 1 through 40 find utility as hybridization probes. In some aspects, the full coding sequence of the transcript, i.e., for SEQ ID NOS. 1-20, are known. Accordingly, any portion of the known sequences available in GenBank, or homologous sequences, can be used in the methods of this invention. It is known in the art that a "perfectly matched" probe is not needed for a specific hybridization. Minor changes in probe sequence achieved by substitution, deletion or insertion of a small number of bases do not affect the hybridization specificity. In general, as much as 20% base-pair mismatch (when optimally aligned) can be tolerated. Preferably, a probe useful for detecting the aforementioned mRNA is at least about 80% identical to the homologous region of comparable size contained in the previously identified sequences identified by SEQ ID NOS. 1 through 20, which correspond to previously characterized genes or SEQ ID NOS. 21-23, 27, 28, 30, 31, 37, 39 and 40, which correspond to known ESTs. More preferably, the probe is 85% identical to the corresponding gene sequence after alignment of the homologous region; even more preferably, it exhibits 90% identity. Percent identity is determined as described above.

These probes can be used in radioassays (e.g. Southern and Northern blot analysis) to detect, prognose, diagnose or monitor various neoplastic states in lung cells or tissue. The probes also can be attached to a solid support such as a chip for use in high throughput screening assays for the detection and monitoring of lung neoplasm. Accordingly, this invention also provides at least one of the transcripts identified as SEQ ID NOS. 1 through 40, or its complement, attached to a solid support for use in high throughput screens.

A polynucleotide of the invention also can be attached to a solid support for use in high throughput screening assays. Using this method, the probes are synthesized on a derivatized glass surface. Photoprotected nucleoside phosphoramidites are coupled to the glass surface, selectively deprotected by photolysis through a photolithographic mask, and reacted with a second protected nucleoside phosphoramidite. The coupling/deprotection process is repeated until the desired probe is complete.

The expression level of a gene is determined through exposure of a nucleic acid sample to the probe-modified chip. Extracted nucleic acid is labeled, for example, with a fluorescent tag, preferably during an amplification step. Hybridization of the labeled sample is performed at an appropriate stringency level. The degree of probe-nucleic acid hybridization is quantitatively measured using a detection device, such as a confocal microscope. See U.S. Patent Nos. 5,578,832; and 5,631,734. The obtained measurement is directly correlated with gene expression level.

Results from the chip assay are typically analyzed using a computer software program. See, for example, EP 717,113 A2 and WO 95/20681. The hybridization data is read into the program, which calculates the expression level of the targeted gene(s). This figure is compared against existing data sets of gene expression levels for that cell type.

For example, the database and methods of using the database provides a means to differentiate a cell expressing a peptide epitope which is the natural counterpart of a synthetic antigenic peptide epitope of the invention from a cell which does not express the epitope or expresses it at a higher or lower level from the cell in question. Expression of polynucleotides encoding the peptide is measured. One cell would serve as a "reference cell" and the cell whose expression of a polynucleotide encoding a peptide corresponding to a synthetic antigenic peptide epitope of the invention is to be measured could be referred to as the "test cell". As an example, the method can be used to distinguish a normal cell (in this case, the reference cell) from a neoplastic cell (i.e., the test cell). It also allows one to differentiate between neoplastic cells biopsied from different regions from a patient or different subjects or gene expression before or after treatment with a potential therapeutic agent. It can be used to analyze drug toxicity and efficacy, as well as to selectively look at protein categories which are expected to be affected by a drug or which may be overexpressed as a result of treatment with a drug, such as the various multi-drug resistant genes. Additional utilities of the database include, but are not limited to analysis of the developmental state of a test cell, the influence of viral or bacterial infection, control of cell cycle, effect of a tumor suppressor gene or lack thereof, polymorphism within the cell type, apoptosis, and the effect of regulatory genes.

The total size of fragment, as well as the size of the complementary stretches, will depend on the intended use or application of the particular nucleic acid segment. Smaller fragments will generally find use in hybridization embodiments, wherein the length of the complementary region may be varied, such as between about 10 and about 100 nucleotides, or even full length according to the complementary sequences one wishes to detect.

Nucleotide probes having complementary sequences over stretches greater than 10 nucleotides in length are generally preferred, so as to increase stability and selectivity of the hybrid, and thereby improving the specificity of particular hybrid molecules obtained. More preferably, one can design nucleic acid molecules having gene-complementary stretches of about 25 nucleotides or more. In some instances, molecules have complementary stretches of about 50 nucleotides in length, or even longer may be desired. Such fragments may be readily prepared by, for example, directly synthesizing the fragment by chemical means, by application of nucleic acid reproduction technology, such as the PCR™ technology with two priming oligonucleotides as described in U.S. Pat. No. 4,603,102 or by introducing selected sequences into recombinant vectors for recombinant production. A preferred probe is about 50-75 or more preferably, 50-100, nucleotides in length.

In certain embodiments, it will be advantageous to employ nucleic acid sequences of the present invention in combination with an appropriate means, such as a label, for detecting hybridization and therefore complementary sequences. A wide variety of appropriate indicator means are known in the art, including fluorescent, radioactive, enzymatic or other ligands, such as avidin/biotin, which are capable of giving a detectable signal. In preferred embodiments, one will likely desire to employ a fluorescent label or an enzyme tag, such as urease, alkaline phosphatase or peroxidase, instead of radioactive or other environmental undesirable reagents. In the case of enzyme tags, colorimetric indicator substrates are known which can be employed to provide a means visible to the human eye or spectrophotometrically, to identify specific hybridization with complementary nucleic acid-containing samples.

Hybridization reactions can be performed under conditions of different "stringency". Relevant conditions include temperature, ionic strength, time of incubation, the presence of additional solutes in the reaction mixture such as formamide, and the washing procedure. Higher stringency conditions are those conditions, such as higher temperature and lower sodium ion concentration, which require higher minimum complementarity between hybridizing elements for a stable hybridization complex to form. Conditions that increase the stringency of a hybridization reaction are widely known and published in the art. See, for example, (Sambrook, *et al.,* (1989), *supra*) and the definitions, *supra.*

The nucleotide probes of the present invention can also be used as primers for the detection of genes or gene transcripts that are expressed in neoplastic lung cells but not normal lung tissue. For the purpose of this invention, amplification means any method employing a primer-dependent polymerase capable of replicating a target sequence with reasonable fidelity. Amplification may be carried out by natural or recombinant DNA-polymerases such as T7 DNA polymerase, Klenow fragment of *E.coli* DNA polymerase, and reverse transcriptase.

A preferred amplification method is PCR. General procedures for PCR are taught in MacPherson, et al. PCR: A PRACTICAL APPROACH (IRL Press at Oxford University Press (1991)). However, PCR conditions used for each application reaction are empirically determined. A number of parameters influence the success of a reaction. Among them are annealing temperature and time, extension time, Mg²⁺ ATP concentration, pH, and the relative concentration of primers, templates, and deoxyribonucleotides.

After amplification, the resulting DNA fragments can be detected by agarose gel electrophoresis followed by visualization with ethidium bromide staining and ultraviolet illumination. A specific amplification polynucleotides having complementary sequences to the polynucleotides identified in SEQ ID NOS. I through 40, demonstrating that the amplified DNA fragment has the predicted size, exhibits the predicated restriction digestion pattern, and/or hybridizes to the correct cloned DNA sequence.

Expression of novel transcript can also be determined by assaying for the presence of the protein product. Determining the protein level involves a) providing a biological sample suspected of containing polypeptides; and (b) measuring the amount of any immunospecific binding that occurs between an antibody reactive to the protein product and detecting the presence of any antibody: protein complex formed. The presence of a complex indicates that the protein product was present in the sample and therefore, the sample contained a neoplastic lung cell.

### Gene Delivery Vehicles and Host Cells

This invention also provides a polynucleotide, as described above, incorporated into a gene delivery vehicle for expression an/or or insertion vector for incorporation into cells. Vectors that contain both a promoter and a cloning site into which a polynucleotide can be operatively linked are well known in the art. Such vectors are capable of transcribing RNA *in vitro* or *in vivo,* and are commercially available from sources such as Stratagene (La Jolla, CA) and Promega Biotech (Madison, WI). In order to optimize expression and/or *in vitro* transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression. Examples of vectors are viruses, such as baculovirus and retrovirus, bacteriophage, adenovirus, adeno-associated virus, cosmid, plasmid, fungal vectors and other recombination vehicles typically used in the art which have been described for expression in a variety of eucaryotic and procaryotic hosts, and may be used for gene therapy as well as for simple protein expression.

Among these are several non-viral vectors, including DNA/liposome complexes, and targeted viral protein DNA complexes. To enhance delivery to a cell, the nucleic acid or proteins of this invention can be conjugated to antibodies or binding fragments thereof which bind cell surface antigens. Liposomes that also comprise a targeting antibody or fragment thereof can be used in the methods of this invention. This invention also provides the targeting complexes for use in the methods disclosed herein.

Polynucleotides are inserted into vector genomes using methods well known in the art. For example, insert and vector DNA can be contacted, under suitable conditions, with a restriction enzyme to create complementary ends on each molecule that can pair with each other and be joined together with a ligase. Alternatively, synthetic nucleic acid linkers can be ligated to the termini of restricted polynucleotide. These synthetic linkers contain nucleic acid sequences that correspond to a particular restriction site in the vector DNA. Additionally, an oligonucleotide containing a termination codon and an appropriate restriction site can be ligated for insertion into a vector containing, for example, some or all of the following: a selectable marker gene, such as the neomycin gene for selection of stable or transient transfectants in mammalian cells; enhancer/promoter sequences from the immediate early gene of human CMV for high levels of transcription; transcription termination and RNA processing signals from SV40 for mRNA stability; SV40 polyoma origins of replication and Co1E1 for proper episomal replication; versatile multiple cloning sites; and T7 and SP6 RNA promoters for *in vitro* transcription of sense and antisense RNA. Other means are well known and available in the art.

This invention further provides host cells, as defined above, comprising a polynucleotide of this invention.

### Polypeptides and Their Utilities

The peptides used in accordance with the methods of the present invention can be obtained in any one of a number of conventional ways. Because they will generally be short sequences, they can be prepared by chemical synthesis using standard techniques. Particularly convenient are the solid phase peptide synthesis techniques. Automated peptide synthesizers are commercially available, as are the reagents required for their use. Alternatively, the peptides can be prepared by enzymatic digestion or cleavage of naturally occurring proteins. The peptides can also be prepared using recombinant techniques known to those of skill in the art.

In one embodiment, isolated peptides of the present invention can be synthesized using an appropriate solid state synthetic procedure. Steward and Young (1968) SOLID PHASE PEPTIDE SYNTHESIS, Freemantle, San Francisco, Calif. A preferred method is the Merrifield process. Merrifield (1967) Recent Progress in Hormone Res. 23:451. The antigenic activity of these peptides may conveniently be tested using, for example, the assays as described herein.

Once an isolated peptide of the invention is obtained, it may be purified by standard methods including chromatography (e.g., ion exchange, affinity, and sizing column chromatography), centrifugation, differential solubility, or by any other standard technique for protein purification. For immunoaffinity chromatography, an epitope may be isolated by binding it to an affinity column comprising antibodies that were raised against that peptide, or a related peptide of the invention, and were affixed to a stationary support.

Alternatively, affinity tags such as hexa-His (Invitrogen), Maltose binding domain (New England Biolabs), influenza coat sequence (Kolodziej et al. (1991) Methods Enzymol. 194:508-509), and glutathione-S-transferase can be attached to the peptides of the invention to allow easy purification by passage over an appropriate affinity column. Isolated peptides can also be physically characterized using such techniques as proteolysis, nuclear magnetic resonance, and x-ray crystallography.

Also included within the scope of the invention are peptides encoded by the polynucleotides of this invention that are differentially modified during or after translation, e.g., by phosphorylation, glycosylation, crosslinking, acylation, proteolytic cleavage, linkage to an antibody molecule, membrane molecule or other ligand. Ferguson et al. (1988) Ann. Rev. Biochem. 57:285-320. This is achieved using various chemical methods or by expressing the polynucleotides in different host cells, e.g., bacterial, mammalian, yeast, or insect cells.

A polypeptide of the invention can be used in a variety of formulations, which may vary depending on the intended use. A polypeptide can be covalently or non-covalently linked (complexed) to various other molecules, the nature of which may vary depending on the particular purpose. For example, a peptide of the invention can be covalently or non-covalently complexed to a macromolecular carrier, including, but not limited to, natural and synthetic polymers, proteins, polysaccharides, poly(aminoacid), polyvinyl alcohol, polyvinyl pyrrolidone, and lipids. A peptide can be conjugated to a fatty acid, for introduction into a liposome. U.S. Patent No. 5,837,249. A synthetic peptide of the invention can be complexed covalently or non-covalently with a solid support, a variety of which are known in the art. A synthetic antigenic peptide epitope of the invention can be associated with an antigen-presenting matrix with or without co-stimulatory molecules, as described in more detail below.

Examples of protein carriers include, but are not limited to, superantigens, serum albumin, tetanus toxoid, ovalbumin, thyroglobulin, myoglobulin, and immunoglobulin.

Peptide-protein carrier polymers may be formed using conventional crosslinking agents such as carbodiimides. Examples of carbodiimides are 1-cyclohexyl-3-(2-morpholinyl-(4-ethyl) carbodiimide (CMC), 1-ethyl-3-(3-dimethyaminopropyl) carbodiimide (EDC) and 1-ethyl-3-(4-azonia-44-dimethylpentyl) carbodiimide.

Examples of other suitable crosslinking agents are cyanogen bromide, glutaraldehyde and succinic anhydride. In general, any of a number of homobifunctional agents including a homobifunctional aldehyde, a homobifunctional epoxide, a homobifunctional imidoester, a homobifunctional N-hydroxysuccinimide ester, a homobifunctional maleimide, a homobifunctional alkyl halide, a homobifunctional pyridyl disulfide, a homobifunctional aryl halide, a homobifunctional hydrazide, a homobifunctional diazonium derivative and a homobifunctional photoreactive compound may be used. Also included are heterobifunctional compounds, for example, compounds having an amine-reactive and a sulfhydryl-reactive group, compounds with an amine-reactive and a photoreactive group and compounds with a carbonyl-reactive and a sulfhydryl-reactive group.

Specific examples of such homobifunctional crosslinking agents include the bifunctional N-hydroxysuccinimide esters dithiobis(succinimidylpropionate), disuccinimidyl suberate, and disuccinimidyl tartarate; the bifunctional imidoesters dimethyl adipimidate, dimethyl pimelimidate, and dimethyl suberimidate; the bifunctional sulfhydryl-reactive crosslinkers 1,4-di-[3'-(2'-pyridyldithio) propion-amido]butane, bismaleimidohexane, and bis-N-maleimido-1, 8-octane; the bifunctional aryl halides 1,5-difluoro-2,4-dinitrobenzene and 4,4'-difluoro-3,3'-dinitrophenylsulfone; bifunctional photoreactive agents such as bis-[b-(4-azidosalicylamido)ethyl]disulfide; the bifunctional aldehydes formaldehyde, malondialdehyde, succinaldehyde, glutaraldehyde, and adipaldehyde; a bifunctional epoxide such as 1,4-butaneodiol diglycidyl ether, the bifunctional hydrazides adipic acid dihydrazide, carbohydrazide, and succinic acid dihydrazide; the bifunctional diazoniums o-tolidine, diazotized and bis-diazotized benzidine; the bifunctional alkylhalides N1N'-ethylene-bis(iodoacetamide), N1N'-hexamethylene-bis(iodoacetamide), NIN' -undecamethylene-bis(iodoacetamide), as well as benzylhalides and halomustards, such as ala'-diiodo-p-xylene sulfonic acid and tri(2-chloroethyl)amine, respectively.

Examples of other common heterobifunctional cross-linking agents that may be used to effect the conjugation of proteins to peptides include, but are not limited to, SMCC succinimidyl-4-(N-maleimidomethyl)cyclohexane-1-carboxylate), MBS (m-maleimidobenzoyl-N-hydroxysuccinimide ester), SIAB (N-succinimidyl(4-iodoacteyl)aminobenzoate), SMPB (succinimidyl-4-(p-maleimidophenyl)butyrate), GMBS (N-(γ-maleimidobutyryloxy)succinimide ester), MPBH (4-(4-N-maleimidopohenyl) butyric acid hydrazide), M2C2H (4-(N-maleimidomethyl) cyclohexane-1-carboxyl-hydrazide), SMPT (succinimidyloxycarbonyl-α-methyl-α-(2-pyridyldithio)toluene), and SPDP (N-succinimidyl 3-(2-pyridyldithio)propionate).

Crosslinking may be accomplished by coupling a carbonyl group to an amine group or to a hydrazide group by reductive amination.

Peptides of the invention also may be formulated as non-covalent attachment of monomers through ionic, adsorptive, or biospecific interactions. Complexes of peptides with highly positively or negatively charged molecules may be done through salt bridge formation under low ionic strength environments, such as in deionized water. Large complexes can be created using charged polymers such as poly-(L-glutamic acid) or poly-(L-lysine) which contain numerous negative and positive charges, respectively. Adsorption of peptides may be done to surfaces such as microparticle latex beads or to other hydrophobic polymers, forming non-covalently associated peptide-superantigen complexes effectively mimicking crosslinked or chemically polymerized protein. Finally, peptides may be non-covalently linked through the use of biospecific interactions between other molecules. For instance, utilization of the strong affinity of biotin for proteins such as avidin or streptavidin or their derivatives could be used to form peptide complexes. These biotin-binding proteins contain four binding sites that can interact with biotin in solution or be covalently attached to another molecule. Wilchek (1988) Anal Biochem. 171:1-32. Peptides can be modified to possess biotin groups using common biotinylation reagents such as the N-hydroxysuccinimidyl ester of D-biotin (NHS-biotin) which reacts with available amine groups on the protein. Biotinylated peptides then can be incubated with avidin or streptavidin to create large complexes. The molecular mass of such polymers can be regulated through careful control of the molar ratio of biotinylated peptide to avidin or streptavidin.

Also provided by this application are the peptides and polypeptides described herein conjugated to a detectable agent for use in the diagnostic methods. For example, detectably labeled peptides and polypeptides can be bound to a column and used for the detection and purification of antibodies. They also are useful as immunogens for the production of antibodies, as described below.

The peptides of this invention also can be combined with various liquid phase carriers, such as sterile or aqueous solutions, pharmaceutically acceptable carriers, suspensions and emulsions. Examples of non-aqueous solvents include propyl ethylene glycol, polyethylene glycol and vegetable oils. When used to prepare antibodies, the carriers also can include an adjuvant that is useful to non-specifically augment a specific immune response. A skilled artisan can easily determine whether an adjuvant is required and select one. However, for the purpose of illustration only, suitable adjuvants include, but are not limited to, Freund's Complete and Incomplete, mineral salts and polynucleotides.

In one embodiment, the peptides of this invention are used to raise antibodies that specifically bind epitopes present on the peptides. The antibodies are useful to detect the gene products isolated from a cell sample.

A variety of techniques are available in the art for protein analysis. They include but are not limited to radioimmunoassays, ELISA (enzyme linked immunoradiometric assays), "sandwich" immunoassays, immunoradiometric assays, in situ immunoassays (using e.g., colloidal gold, enzyme or radioisotope labels), western blot analysis, immunoprecipitation assays, immunoflourescent assays, and PAGE-SDS. See Harlow and Lane (1988) *supra.* and Sambrook et al. (1989) *supra.*

To detect and quantify the immunospecific binding, or signals generated during hybridization or amplification procedures, digital image analysis systems including but not limited to those that detect radioactivity of the probes or chemiluminescence can be employed.

In diagnosing malignancy, hyperplasia or metaplasia characterized by a expression of a transcript identified herein by SEQ ID NOS. 1 through 40, or its complement, one typically conducts a comparative analysis of the subject and appropriate controls. Preferably, a diagnostic test includes a control sample derived from a subject (hereinafter positive control), that exhibits expression of the transcript and clinical characteristics of the malignancy or metaplasia lung cancer. More preferably, a diagnosis also includes a control sample derived from a subject (hereinafter negative control), that lacks the clinical characteristics of the neoplastic state and whose expression of the transcript in question is negative or *de minimus.* A positive correlation between the subject and the positive control with respect to the identified alterations indicates the presence or a predisposition to the development of a neoplastic lung condition. A lack of correlation between the subject and the negative control confirms the diagnosis.

### Drug Screening

The present invention also provides a screen for various agents and methods for reversing the neoplastic condition of the cells or selectively inhibiting growth or proliferation of the cells described above. In one aspect, the screen assays for agents which are useful for the treatment of malignancy, hyperplasia or metaplasia characterized by expression of the transcript described herein.

Thus, to practice the method *in vitro,* suitable cell cultures or tissue cultures are first provided. The cell can be a cultured cell or a genetically modified cell in which a transcript from SEQ ID NOS. 1 through 40, or its complement, is expressed. Alternatively, the cells can be from a tissue biopsy. The cells are cultured under conditions (temperature, growth or culture medium and gas (CO₂)) and for an appropriate amount of time to attain exponential proliferation without density dependent constraints. It also is desirable to maintain an additional separate cell culture; one which does not receive the agent being tested as a control.

As is apparent to one of skill in the art, suitable cells may be cultured in microtiter plates and several agents may be assayed at the same time by noting genotypic changes, phenotypic changes or cell death.

When the agent is a composition other than a DNA or RNA nucleic acid molecule, the suitable conditions may be by directly added to the cell culture or added to culture medium for addition. As is apparent to those skilled in the art, an "effective" amount must be added which can be empirically determined.

For the purposes of this invention, an "agent" is intended to include, but not be limited to a biological or chemical compound such as a simple or complex organic or inorganic molecule, a peptide, a protein (e.g. antibody) or an oligonucleotide (e.g. anti-sense). A vast array of compounds can be synthesized, for example oligomers, such as oligopeptides and oligonucleotides, and synthetic organic compounds based on various core structures, and these are also included in the term "agent". In addition, various natural sources can provide compounds for screening, such as plant or animal extracts, and the like. It should be understood, although not always explicitly stated that the agent is used alone or in combination with another agent, having the same or different biological activity as the agents identified by the inventive screen. The agents and methods also are intended to be combined with other therapies.

As noted above, lung cells having overexpression of a proto-oncogene that results in the neoplastic state are suitably treated by this method. These cells can be identified by any method known in the art that allows for the identification of overexpression of the proto-oncogene. One such method is exemplified below.

When the agent is a nucleic acid, it can be added to the cell cultures by methods well known in the art, which includes, but is not limited to calcium phosphate precipitation, microinjection or electroporation.

One can determine if the object of the method, *i.e.,* reversal of the neoplastic state of the cell, has been achieved by a reduction of cell division, differentiation of the cell or assaying for a reduction or loss of transcript expression. Cellular differentiation can be monitored by histological methods or by monitoring for the presence or loss of certain cell surface markers, which may be associated with an undifferentiated phenotype, *e.g.* CD34 on primative hematopoietic stem cells..

Kits containing the agents and instructions necessary to perform the screen and *in vitro* method as described herein also are claimed.

When the subject is an animal such as a rat or mouse, the method provides a convenient animal model system which can be used prior to clinical testing of the therapeutic agent. In this system, a candidate agent is a potential drug if transcript expression is reduced in a neoplastic lung cell or if symptoms associated or correlated to the presence of cells containing transcript expression are ameliorated, each as compared to untreated, animal having the pathological cells. It also can be useful to have a separate negative control group of cells or animals which are healthy and not treated, which provides a basis for comparison.

These agents of this invention and the above noted compounds and their derivatives may be used for the preparation of medicaments for use in the methods described herein.

In a preferred embodiment, an agent of the invention is administered to treat lung cancer. In a further preferred embodiment, an agent of the invention is administered to treat squamous cell lung cancer. Therapeutics of the invention can also be used to prevent progression from a pre-neoplastic or non-malignant state into a neoplastic or a malignant state.

Various delivery systems are known and can be used to administer a therapeutic agent of the invention, e.g., encapsulation in liposomes, microparticles, microcapsules, expression by recombinant cells, receptor-mediated endocytosis (see, *e.g.*, Wu and Wu (1987) J. Biol. Chem. 262:4429-4432), construction of a therapeutic nucleic acid as part of a retroviral or other vector, etc. Methods of delivery include but are not limited to intra-arterial, intramuscular, intravenous, intranasal, and oral routes. In a specific embodiment, it may be desirable to administer the pharmaceutical compositions of the invention locally to the area in need of treatment; this may be achieved by, for example, and not by way of limitation, local infusion during surgery, by injection, or by means of a catheter.

The agents identified herein as effective for their intended purpose can be administered to subjects or individuals susceptible to or at risk of developing a disease correlated to the overexpression of these proto-oncogenes. When the agent is administered to a subject such as a mouse, a rat or a human patient, the agent can be added to a pharmaceutically acceptable carrier and systemically or topically administered to the subject. To determine patients that can be beneficially treated, a tumor sample is removed from the patient and the cells are assayed for the overexpression of the proto-oncogene. Therapeutic amounts can be empirically determined and will vary with the pathology being treated, the subject being treated and the efficacy and toxicity of the agent. When delivered to an animal, the method is useful to further confirm efficacy of the agent. As an example of an animal model, groups of nude mice (Balb/c NCR nu/nu female, Simonsen, Gilroy, CA) are each subcutaneously inoculated with about 10⁵ to about 10⁹ hyperproliferative, cancer or target cells as defined herein. When the tumor is established, the agent is administered, for example, by subcutaneous injection around the tumor. Tumor measurements to determine reduction of tumor size are made in two dimensions using venier calipers twice a week. Other animal models may also be employed as appropriate.

Administration *in vivo* can be effected in one dose, continuously or intermittently throughout the course of treatment. Methods of determining the most effective means and dosage of administration are well known to those of skill in the art and will vary with the composition used for therapy, the purpose of the therapy, the target cell being treated, and the subject being treated. Single or multiple administrations can be carried out with the dose level and pattern being selected by the treating physician. Suitable dosage formulations and methods of administering the agents can be found below.

The agents and compositions of the present invention can be used in the manufacture of medicaments and for the treatment of humans and other animals by administration in accordance with conventional procedures, such as an active ingredient in pharmaceutical compositions.

The pharmaceutical compositions can be administered orally, intranasally, parenterally or by inhalation therapy, and may take the form of tablets, lozenges, granules, capsules, pills, ampoules, suppositories or aerosol form. They may also take the form of suspensions, solutions and emulsions of the active ingredient in aqueous or nonaqueous diluents, syrups, granulates or powders. In addition to an agent of the present invention, the pharmaceutical compositions can also contain other pharmaceutically active compounds or a plurality of compounds of the invention.

More particularly, an agent of the present invention also referred to herein as the active ingredient, may be administered for therapy by any suitable route including oral, rectal, nasal, topical (including transdermal, aerosol, buccal and sublingual), vaginal, parental (including subcutaneous, intramuscular, intravenous and intradermal) and pulmonary. It will also be appreciated that the preferred route will vary with the condition and age of the recipient, and the disease being treated.

Ideally, the agent should be administered to achieve peak concentrations of the active compound at sites of disease. This may be achieved, for example, by the intravenous injection of the agent, optionally in saline, or orally administered, for example, as a tablet, capsule or syrup containing the active ingredient. Desirable blood levels of the agent may be maintained by a continuous infusion to provide a therapeutic amount of the active ingredient within disease tissue. The use of operative combinations is contemplated to provide therapeutic combinations requiring a lower total dosage of each component antiviral agent than may be required when each individual therapeutic compound or drug is used alone, thereby reducing adverse effects.

While it is possible for the agent to be administered alone, it is preferable to present it as a pharmaceutical formulation comprising at least one active ingredient, as defined above, together with one or more pharmaceutically acceptable carriers therefor and optionally other therapeutic agents. Each carrier must be "acceptable" in the sense of being compatible with the other ingredients of the formulation and not injurious to the patient.

Formulations include those suitable for oral, rectal, nasal, topical (including transdermal, buccal and sublingual), vaginal, parenteral (including subcutaneous, intramuscular, intravenous and intradermal) and pulmonary administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets, each containing a predetermined amount of the active ingredient; as a powder or granules; as a solution or suspension in an aqueous or non-aqueous liquid; or as an oil-in-water liquid emulsion or a water-in-oil liquid emulsion. The active ingredient may also be presented a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder (e.g., povidone, gelatin, hydroxypropylmethyl cellulose), lubricant, inert diluent, preservative, disintegrant (*e.g.*, sodium starch glycolate, cross-linked povidone, cross-linked sodium carboxymethyl cellulose) surface-active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Pharmaceutical compositions for topical administration according to the present invention may be formulated as an ointment, cream, suspension, lotion, powder, solution, past, gel, spray, aerosol or oil. Alternatively, a formulation may comprise a patch or a dressing such as a bandage or adhesive plaster impregnated with active ingredients and optionally one or more excipients or diluents.

If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the agent through the skin or other affected areas. Examples of such dermal penetration enhancers include dimethylsulfoxide and related analogues.

The oily phase of the emulsions of this invention may be constituted from known ingredients in an known manner. While this phase may comprise merely an emulsifier (otherwise known as an emulgent), it desirably comprises a mixture of at lease one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and/or fat make up the so-called emulsifying ointment base which forms the oily dispersed phase of the cream formulations.

Emulgents and emulsion stabilizers suitable for use in the formulation of the present invention include Tween 60, Span 80, cetostearyl alcohol, myristyl alcohol, glyceryl monostearate and sodium lauryl sulphate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the agent.

Formulations for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the agent, such carriers as are known in the art to be appropriate.

Formulations suitable for nasal administration, wherein the carrier is a solid, include a coarse powder having a particle size, for example, in the range of about 20 to about 500 microns which is administered in the manner in which snuff is taken, i.e., by rapid inhalation through the nasal passage from a container of the powder held close up to the nose. Suitable formulations wherein the carrier is a liquid for administration as, for example, nasal spray, nasal drops, or by aerosol administration by nebulizer, include aqueous or oily solutions of the agent.

Formulations suitable for parenteral administration include aqueous and non-aqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily subdose, as herein above-recited, or an appropriate fraction thereof, of a agent.

It should be understood that in addition to the ingredients particularly mentioned above, the formulations of this invention may include other agents conventional in the art having regard to the type of formulation in question, for example, those suitable for oral administration may include such further agents as sweeteners, thickeners and flavoring agents. It also is intended that the agents, compositions and methods of this invention be combined with other suitable compositions and therapies.

### Antibodies

Also provided by this invention is an antibody capable of specifically forming a complex with the proteins or polypeptides as described above. The term "antibody" includes polyclonal antibodies and monoclonal antibodies. The antibodies include, but are not limited to mouse, rat, and rabbit or human antibodies.

Laboratory methods for producing polyclonal antibodies and monoclonal antibodies, as well as deducing their corresponding nucleic acid sequences, are known in the art, see Harlow and Lane (1988) *supra* and Sambrook et al. (1989) *supra.* The monoclonal antibodies of this invention can be biologically produced by introducing protein or a fragment thereof into an animal, e.g., a mouse or a rabbit. The antibody producing cells in the animal are isolated and fused with myeloma cells or heteromyeloma cells to produce hybrid cells or hybridomas. Accordingly, the hybridoma cells producing the monoclonal antibodies of this invention also are provided.

Thus, using the protein or fragment thereof, and well known methods, one of skill in the art can produce and screen the hybridoma cells and antibodies of this invention for antibodies having the ability to bind the proteins or polypeptides.

If a monoclonal antibody being tested binds with the protein or polypeptide, then the antibody being tested and the antibodies provided by the hybridomas of this invention are equivalent. It also is possible to determine without undue experimentation, whether an antibody has the same specificity as the monoclonal antibody of this invention by determining whether the antibody being tested prevents a monoclonal antibody of this invention from binding the protein or polypeptide with which the monoclonal antibody is normally reactive. If the antibody being tested competes with the monoclonal antibody of the invention as shown by a decrease in binding by the monoclonal antibody of this invention, then it is likely that the two antibodies bind to the same or a closely related epitope. Alternatively, one can pre-incubate the monoclonal antibody of this invention with a protein with which it is normally reactive, and determine if the monoclonal antibody being tested is inhibited in its ability to bind the antigen. If the monoclonal antibody being tested is inhibited then, in all likelihood, it has the same, or a closely related, epitopic specificity as the monoclonal antibody of this invention.

The term "antibody" also is intended to include antibodies of all isotypes. Particular isotypes of a monoclonal antibody can be prepared either directly by selecting from the initial fusion, or prepared secondarily, from a parental hybridoma secreting a monoclonal antibody of different isotype by using the sib selection technique to isolate class switch variants using the procedure described in Steplewski et al. (1985) Proc. Natl. Acad. Sci. 82:8653 or Spira et al. (1984) J. Immunol. Methods 74:307.

This invention also provides biological active fragments of the polyclonal and monoclonal antibodies described above. These "antibody fragments" retain some ability to selectively bind with its antigen or immunogen. Such antibody fragments can include, but are not limited to:
(1) Fab,
(2) Fab',
(3) F(ab')₂,
(4) Fv, and
(5) SCA

A specific example of "a biologically active antibody fragment" is a CDR region of the antibody. Methods of making these fragments are known in the art, see for example, Harlow and Lane (1988) *supra.*

The antibodies of this invention also can be modified to create chimeric antibodies and humanized antibodies (Oi, et al. (1986) BioTechniques 4(3):214). Chimeric antibodies are those in which the various domains of the antibodies' heavy and light chains are coded for by DNA from more than one species.

The isolation of other hybridomas secreting monoclonal antibodies with the specificity of the monoclonal antibodies of the invention can also be accomplished by one of ordinary skill in the art by producing anti-idiotypic antibodies (Herlyn et al. (1986) Science 232:100). An anti-idiotypic antibody is an antibody which recognizes unique determinants present on the monoclonal antibody produced by the hybridoma of interest.

Idiotypic identity between monoclonal antibodies of two hybridomas demonstrates that the two monoclonal antibodies are the same with respect to their recognition of the same epitopic determinant. Thus, by using antibodies to the epitopic determinants on a monoclonal antibody it is possible to identify other hybridomas expressing monoclonal antibodies of the same epitopic specificity.

It is also possible to use the anti-idiotype technology to produce monoclonal antibodies which mimic an epitope. For example, an anti-idiotypic monoclonal antibody made to a first monoclonal antibody will have a binding domain in the hypervariable region which is the mirror image of the epitope bound by the first monoclonal antibody. Thus, in this instance, the anti-idiotypic monoclonal antibody could be used for immunization for production of these antibodies.

As used in this invention, the term "epitope" is meant to include any determinant having specific affinity for the monoclonal antibodies of the invention. Epitopic determinants usually consist of chemically active surface groupings of molecules such as amino acids or sugar side chains and usually have specific three dimensional structural characteristics, as well as specific charge characteristics.

The antibodies of this invention can be linked to a detectable agent or label. There are many different labels and methods of labeling known to those of ordinary skill in the art.

The antibody-label complex is useful to detect the protein or fragments in a sample, using standard immunochemical techniques such as immunohistochemistry as described by Harlow and Lane (1988) *supra.* Competitive and non-competitive immunoassays in either a direct or indirect format are examples of such assays, e.g., enzyme linked immunoassay (ELISA) radioimmunoassay (RIA) and the sandwich (immunometric) assay. Those of skill in the art will know, or can readily discern, other immunoassay formats without undue experimentation.

The coupling of antibodies to low molecular weight haptens can increase the sensitivity of the assay. The haptens can then be specifically detected by means of a second reaction. For example, it is common to use haptens such as biotin, which reacts avidin, or dinitropherryl, pyridoxal, and fluorescein, which can react with specific anti-hapten antibodies. See Harlow and Lane (1988) *supra.*

The monoclonal antibodies of the invention also can be bound to many different carriers. Thus, this invention also provides compositions containing the antibodies and another substance, active or inert. Examples of well-known carriers include glass, polystyrene, polypropylene, polyethylene, dextran, nylon, amylases, natural and modified celluloses, polyacrylamides, agaroses and magnetite. The nature of the carrier can be either soluble or insoluble for purposes of the invention. Those skilled in the art will know of other suitable carriers for binding monoclonal antibodies, or will be able to ascertain such, using routine experimentation.

### Compositions

Also provided by this invention are compositions containing or comprising one or more of the polynucleotides, genes, polypeptides, proteins, gene delivery vehicles, vectors, host cells or antibodies and a carrier, such as a solid support. In one embodiment, the carrier is a pharmaceutically acceptable carrier, as defined above. Medicaments useful for the diagnosis and treatment of lung cancer are further provided by this invention.

### Non-Human Transgenic Animals

In another aspect, the novel polynucleotide sequences associated with lung cancer can be used to generate transgenic animal models. In recent years, geneticists have succeeded in creating transgenic animals, for example mice, by manipulating the genes of developing embryos and introducing foreign genes into these embryos. Once these genes have integrated into the genome of the recipient embryo, the resulting embryos or adult animals can be analyzed to determine the function of the gene. The mutant animals are produced to understand the function of known genes *in vivo* and to create animal models of human diseases. (*see, e.g.,* Chisaka *et al.* (1992) 355:516-520; Joyner et al. (1992) in POSTIMPLANTATION DEVELOPMENT IN THE MOUSE (Chadwick and Marsh, eds., John Wiley & Sons, United Kingdom) pp:277-297; Dorin et al. (1992) Nature 359:211-215).

U.S. Patent Nos. 5,464,764 and 5,487,992 describe one type of transgenic animal in which the gene of interest is deleted or mutated sufficiently to disrupt its function. (*See, also* U.S. Patent Nos. 5,631,153 and 5,627,059). These "knockout" animals, made by taking advantage of the phenomena of homologous recombination, can be used to study the function of a particular gene sequence *in vivo*. The polynucleotide sequences described herein are useful in preparing animal models of lung cancer.

### Computational Analysis and Genomics Applications

This invention also provides a process for preparing a database comprising at least one of the sequences identified in SEQ ID NO. 1 to 40 or its respective complement. Alternatively, the database comprises at least one of the sequences selected from the group consisting of 24 to 26, 29, 32, or 38, or its respective complement. The polynucleotide sequences are stored in a digital storage medium such that a data processing system for standardized representation of the genes that identify a lung cancer cell is compiled. The data processing system is useful to analyze gene expression between two cells by first selecting a cell suspected of being of a neoplastic phenotype or genotype and then isolating polynucleotides from the cell. The isolated polynucleotides are sequenced. The sequences from the sample are compared with the sequence(s) present in the database using homology search techniques. Greater than 90%, more preferably greater than 95% and more preferably, greater than or equal to 97% sequence identity between the test sequence and at least one sequence identified by SEQ ID NO. 1 through 40 or its complement is a positive indication that the polynucleotide has been isolated from a lung cancer cell as defined above.

In an alternative embodiment, the polynucleotides of this invention are sequenced and the information regarding sequence and in some embodiments, relative expression, is stored in any functionally relevant program, e.g., in Compare Report using the SAGE software (available through Dr. Ken Kinzler at Johns Hopkins University). The Compare Report provides a tabulation of the polynucleotide sequences and their abundance for the samples (say A, B, C and D above) normalized to a defined number of polynucleotides per library (say 25,000). This is then imported into MS-ACCESS either directly or via copying the data into an Excel spreadsheet first and then from there into MS-ACCESS for additional manipulations. Other programs such as SYBASE or Oracle that permit the comparison of polynucleotide numbers could be used as alternatives to MS-ACCESS. Enhancements to the software can be designed to incorporate these additional functions. These functions consist in standard Boolean, algebraic, and text search operations, applied in various combinations to reduce a large input set of polynucleotides to a manageable subset of polynucleotides of specifically defined interest.

The researcher may create groups containing one or more project(s) by combining the counts of specific polynucleotides within a group (e.g., GroupNormal = Normal1 + Normal2, GroupTumor = Primary Tumor1 + TumorCellLine). Additional characteristic values are also calculated for each tag in the group (e.g., average count, minimum count, maximum count). The researcher may calculate individual tag count ratios between groups, for example the ratio of the average GroupNormal count to the average GroupTumor count for each polynucleotide. The researcher may calculate a statistical measure of the significance of observed differences in tag counts between groups.

The following examples are intended to illustrate, but not limit this invention.

### EXAMPLES

### Example 1:

A systematic analysis of transcripts present in non-small cell lung cancer (NSCLC) was performed by Serial Analysis of Gene Expression ("SAGE") (U.S. Patent No. 5,695,937). Primary squamous cell lung cancers containing over 95% neoplastic components from two unrelated patients were selected for SAGE analysis. Patient A was 58-year old and diagnosed with moderately differentiated cancer at the lower right lobe of the lung at the time of surgery. Patient B was 68-year old and diagnosed with poorly differentiated cancer of the lower right lobe. Normal small airway epithelial cells obtained from two independent individuals were used as the negative controls.

The SAGE libraries were constructed essentially as described in Velculescu V et al. (1995) Science 270:484-487. PolyA RNAs isolated from lung tumors of patients A and B, and from normal small airway epithelial cells were converted to double-stranded cDNA. The cDNA was then cleaved with an anchoring enzyme NlaIII and divided into two pools. Linkers containing recognition sites for the tagging enzyme BsmFI was ligated to each pool: After BsmF1 restriction, SAGE tag overhangs were filled-in with Klenow, and tags from the two pools were combined and ligated to each other. The ligation product was diluted and then amplified by PCR. The resulting PCR product was then analyzed by polyacrylamide gel electrophoresis (PAGE), and the PCR product containing two tags ligated tail to tail (ditag) was excised and then cleaved with NlaIII. After NlaIII restriction, the ditags was excised and self-ligated. The concatenated products were separated by PAGE and those containing ~500 to 2000 nucleotide base pairs were excised and cloned for subsequent sequence analysis.

Approximately 2000-4000 individual colonies were isolated and sequenced from each SAGE library. The sequences of over 50,000 tags that represent about 15,000 unique transcripts in each library were analyzed in order to generate a comprehensive profile of gene expression patterns in lung cancer. Tables I and II summarize the comparative SAGE analyses of cDNA clones derived from the lung cancers of two individuals and the lungs of two normal individuals.

Table I provides the transcripts of those sequences which correspond to previously characterized genes.

**TABLE I**

| **Sequence** | **Known Gene** | **SEQ ID NO** | **GenBank Accession No.** |
|---|---|---|---|
| AAGGAGCAAG | carboxylesterase | 1 | X52973; M5509 |
| CTCCTGGGCG | NB1 | 2 | M58026; AA0555 |
| GATAGCACAG | 1GFbp5 | 3 | L27560 |
| TGCTGCCTGT | HCG4, BST2 | 4 | X81005 |
| CCATTTTTAC | U2snrnp aux fac | 5 | M96982 |
| GTCCCTGCCT | GST sub 4 or GST 1 or GSTM 2 | 6 | X08020; J03817 |
| CAACTAATTC | apolipe J or SP40 or trpm-2, or sulfated gp2 | 7 | X14723; J02908; M74816 |
| GTTATAAGAT | DSS1 | 8 | U41515; U61847 |
| TATTTTTGTT | thioredoxin reductase | 9 | X91247; S79851 |
| CAGATAACAT | myeloblast mitochondrial outer memb protein | 10 | D13641 |
| TGTACCTGTA | α-tubulin | 11 | K00558 |
| CCAGGGGAGA | p27 | 12 | X67325 |
| GAGAAAACCC ATGTACCTGA | sox 2 or HMG box | 13 | Z31560 |
| | epithelial memb proT 2/XMP | 14 | X94770; U52100 |
| TTCTAACATA | Na/K ATPase β subunit | 15 | X03747; U16799; M25160 |
| GGTGGTGTCT | glutathione perox-like protein | 16 | X68314; X53463 |
| TACTAGTCCT | HSP90 | 17 | D87666; X15183 |
| ATGCAGCCAT | ODC-1 | 18 | X55362 |
| TGCTGCCCTG | B-myb | 19 | X13293; T28987 |
| TGGCCCGACG | 8-oxo-D-GTPase | 20 | D16581 |

Table II summarizes those trancripts that do not correspond to a previously characterized gene. Several of the sequences correspond to reported Expressed Sequence Tags (ESTs). Based on the inventors search of publicly available databases, the remaining transcripts identify novel, uncharacterized genes.

**TABLE II**

| **Sequence** | **EST (Yes/No)** | **SEQ ID NO** | **GenBank Accession No.** |
|---|---|---|---|
| TGCCGTTTTG | Yes | 21 | AA1272 |
| GATGAGGAGA | Yes | 22 | AA0228; AA0372 |
| TGGAAATGAC | Yes | 23 | AA0451 |
| TAATACTTTT | No | 24 | None |
| CAATAAAATT | No | 25 | None |
| AAGGCTGGAA | No | 26 | None |
| CGGCCACAGA | Yes | 27 | AA1142; AA1215 |
| GCGCAGACTT | Yes | 28 | R87082 |
| TATACGCTCA | No | 29 | None |
| TAGTAAGTCA | Yes | 30 | AA1220 |
| GCTTGAATAA | Yes | 31 | H88507 |
| TCCCCGTTAC | No | 32 | None |
| ACCTTTACTG | No | 33 | None |
| TCCCCGTAAC | No | 34 | None |
| ATGATCCCTG | No | 35 | None |
| TATCTGTCTA | Yes | 36 | AA0635 |
| TCTGCTAAAG | Yes | 37 | AA0294; AA0456 |
| TCCCTAATTA | No | 38 | None |
| GAATCTGGAG | Yes | 39 | S53724; AA0019 |
| GACGACTGAC | Yes | 40 | N29050; W24362 |

### Cloning and Sequencing of Genes Corresponding to Novel Transcripts

This invention also provides polynucleotides which are fragments of novel, uncharacterized genes. These transcripts are provided in SEQ ID NOS. 24-26, 29, 32-35 and 38. Using the methods disclosed herein, the open reading frames of the genes corresponding to these transcripts can be isolated and sequenced. Accordingly, polynucleotides comprising these transcripts or their respective complements also are provided by this invention. The complete open reading frames or fragments thereof can be inserted into a vector or host cell and used to reproduce the sequences or produce polypeptides. Antibodies, and in particular, monoclonal antibodies, that specifically bind to the polypeptides may be generated based on well known methods. The antibodies can be used to screen for expression of the gene corresponding to the transcript.

It is to be understood that while the invention has been described in conjunction with the above embodiments, that the foregoing description and examples are intended to illustrate and not limit the scope of the invention. Other aspects, advantages and modifications within the scope of the invention will be apparent to those skilled in the art to which the invention pertains.
1. A population of polynucleotides comprising at least one polynucleotide comprising a polynucleotide sequence selected from the group consisting of SEQ ID NOS. 1 through 40, or its respective complement.
2. The population of embodiment 1, wherein the one polynucleotide comprises a novel tag or its complement selected from the group consisting of SEQ ID NOS. 24 to 26, 29, 32 to 35 or 38, or its respective complement.
3. An isolated polynucleotide comprising a polynucleotide sequence selected from the group of sequences consisting of SEQ ID NOS: 24 to 26, 29, 32 to 35 or 38, or its respective complement.
4. A gene delivery vehicle comprising an isolated polynucleotide of any of embodiments 1 to 3.
5. A host cell comprising an isolated polynucleotide of any of embodiments 1 to 3.
6. An isolated polynucleotide comprising a polynucleotide sequence obtained by identification of larger fragment or full-length coding sequence of the sequence depicted in SEQ ID NOS: 24 to 26, 29, 32 to 35 or 38, or its respective complement.
7. An isolated second polynucleotide corresponding to the polynucleotide of embodiment 1.
8. An isolated polynucleotide fragment of the polynucleotide of embodiment 7.
9. A polynucleotide according to embodiment 6 attached to a solid support.
10. A polynucleotide comprising a polynucleotide sequence selected from the group consisting of SEQ ID NOS: 24 to 26, 29, 32 to 35 or 38, or its respective complement attached to a solid support.
11. The polynucleotide of embodiment 10, wherein the solid support is a chip array.
12. An isolated polypeptide comprising a polypeptide encoded by the polynucleotide sequence of embodiment 6.
13. An antibody which recognizes an epitope on a polypeptide of embodiment 12.
14. A method for detecting a lung cancer cell, comprising contacting a polynucleotide isolated from a sample suspected of containing the lung cancer cell with a polynucleotide selected from the group consisting of SEQ ID NOS: 1 through 40, under conditions that favor hybridization of complementary polynucleotides an detecting a hybridized complement, wherein overexpression of the hybridized complement is indicative of the presence of a lung cancer cell.
15. A method for detecting a lung cancer cell, comprising contacting a polynucleotide isolated from a sample suspected of containing the lung cancer cell with a polynucleotide obtained by identification of larger fragment or full-length coding sequences of the sequences selected from the group consisting of SEQ ID NOS: 1 through 40, under conditions that favor hybridization of complementary polynucleotides and detecting a hybridized complement, wherein overexpression of the hybridized complement is indicative of the presence of a lung cancer cell.
16. The method of embodiment 14 or 15, wherein the polynucleotide is immobilized on a solid support.
17. A method for detecting a lung cancer cell, comprising contacting a polynucleotide isolated from a sample suspected of containing the lung cancer cell with a polynucleotide selected from the group consisting of polynucleotides of SEQ ID NOS. 1 through 40, under conditions that favor hybridization; and amplifying complementary polynucleotides in the sample, wherein detection the amplified polynucleotides is indicative of a lung cancer cell.
18. A method for detecting a lung cancer cell, comprising contacting a polynucleotide isolated from a sample suspected of containing the lung cancer cell with a polynucleotide obtained by identification or larger fragment or full-length coding sequences of the polynucleotide sequences selected from the group consisting of polynucleotides of SEQ ID NOS. 1 through 40, under conditions that favor hybridization; and amplifying complementary polynucleotides in the sample, wherein detection the amplified polynucleotides is indicative of a lung cancer cell.
19. A method for detecting a lung cancer cell, comprising contacting a sample suspected of containing the lung cancer cell an agent that specifically binds to a gene product produced from a gene selected from the group consisting of carboxylesterase, NB1, 1GFbp5, HCG4, BST2, U2snrnp aux fac, 8-oxo-D-GTPase, GST sub 4 or GST 1 or GSTM 2, apolipe J or SP40 or trpm-2, or sulfated gp2, DSS1, thioredoxin reductase, B-myb, myeloblast mitochondrial outer memb protein, α-tubulin, p27, sox 2 or HMG box, epithelial memb proT 2/XMP, Na/K ATPase β subunit, glutathione perox-like protein, HSP90, and ODC-1, and detecting any agent:gene product complex so formed, thereby detecting a lung cancer cell.
20. The method of embodiment 18, wherein the agent is a monoclonal antibody.
21. A method for detecting a lung cancer cell, comprising contacting a sample suspected of containing the lung cancer cell with an agent that specifically binds to a gene product produced from a polynucleotide comprising a polynucleotide sequence obtained by identification of larger fragment or full-length coding sequence selected from the group consisting of SEQ ID NOS: 24 to 26, 29, 32 to 35 or 38, and detecting any agent:peptide complex so formed, thereby detecting a lung cancer cell.
22. The method for embodiment 21, wherein the agent is a monoclonal antibody.
23. A system for identifying selected polynucleotide records that identify a lung cancer cell, the system comprising:
   a digital computer;
   a database coupled to the computer
   a database coupled to the database server having data stored therein, the data comprising records of data combined from polynucleotide obtained from the polynucleotide sequences comprising SEQ ID NOS: 1 to 40; and
   a code mechanism for applying queries based upon a desired selection criteria to the data file in the database to produce reports of polynucleotide records which match the desired selection criteria.
24. A method for detecting a lung cancer cell, using a computer having a processor, memory, display, and input/output devices, the method comprising the steps of:
   a) providing a sequence of a polynucleotide isolated from a sample suspected of containing a lung cancer cell;
   b) providing the database of embodiment 23; and
   c) using a code mechanism for applying queries based upon a desired selection criteria to the data file in the database to produce reports of polynucleotide records of step a) which provide a match of the desired selection criteria of the sequences in the database of step b), the presence of a match being a positive indication that the polynucleotide of step a) has been isolated from a cell that is a lung cancer cell.
25. A screen for polynucleotides differentially expressed in lung cancer cells, comprising searching sequence databases for nucleotide sequences homologous to a polynucleotide selected from the group consisting of SEQ ID NOS: 1 through 40 and detecting sequences with homology thereby identifying polynucleotides differentially expressed in lung cancer cells.
26. A screen for a potential therapeutic agent for the reversal of the neoplastic condition of a lung cell wherein the cell is characterized by expression of a polynucleotide selected from the group consisting of the polynucleotides depicted in SEQ ID NOS. 1 through 40, comprising contacting a cell expressing the polynucleotide with an effective amount of a potential agent and assaying for reversal of the neoplastic condition.
27. A screen for a potential therapeutic agent for the reversal of the neoplastic condition of a lung cell wherein the cell is characterized by expression of a polynucleotide selected from the group consisting of the polynucleotides obtained by identification or larger fragment or full-length coding sequences of the sequences depicted in SEQ ID NOS: 1 to 40, comprising contacting a cell expressing the polynucleotide with an effective amount of a potential agent and assaying for reversal of the neoplastic condition.
28. A kit use in a detection method according to any one of embodiments 11 to 15 comprising in suitable packaging:
   one or more polynucleotides selected from the group consisting of SEQ ID NOS: 1-40 immobilized on a solid support; and
   a reagent suitable for hybridizing a sample suspected of containing the lung cancer cell.
29. A non-human transgenic animal having a disruption in a polynucleotide corresponding to a polynucleotide having a sequence selected from the group consisting of SEQ ID NOS: 1-40

## Claims

1. A method for detecting a lung cancer cell, comprising contacting a polynucleotide isolated from a sample suspected of containing the lung cancer cell with a polynucleotide comprising SEQ ID NO:16, wherein SEQ ID NO:16 is adjoined at the 5' end to a NlaIII restriction enzyme site, under conditions that favor hybridization of complementary polynucleotides and detecting a hybridized complement, and wherein overexpression of the hybridized complement is indicative of the presence of a lung cancer cell, or under conditions that favor hybridization and amplifying complementary polynucleotides in the sample, and wherein detection of the amplified polynucleotides is indicative of a lung cancer cell.

2. Method according to claim 1 under conditions that favor hybridization of complementary polynucleotides and detecting a hybridized complement, wherein the polynucleotide is immobilized on a solid support.

3. A method for detecting a lung cancer cell, comprising contacting a sample suspected of containing the lung cancer cell with an antibody or oligonucleotide that specifically binds to a gene product produced from a polynucleotide, wherein said polynucleotide comprises a SAGE tag as shown in SEQ ID NO:16 wherein the SAGE tag is adjoined at the 5' end to a NlaIII restriction enzyme site and detecting any antibody:gene product complex or oligonucleotide:gene product complex so formed, thereby detecting a lung cancer cell.

4. Method according to claim 3, comprising: contacting the sample suspected of containing the lung cancer cell with the antibody or oligonucleotide that specifically binds to the gene product produced from a polynucleotide over-represented in the lung cancer cell and not in the normal lung cell, and detecting any antibody:gene product complexes or oligonucleotide:gene product complexes so formed wherein the presence of said complex indicates the presence of a cell expressing said gene product, thereby detecting a lung cancer cell in said sample.

5. Method according to claim 4, comprising contacting a control sample not containing the lung cancer cell with the antibody or oligonucleotide and detecting any antibody:gene product complexes or oligonucleotide:gene product complexes so formed wherein the presence of said complex in the sample but not in the control sample indicates the presence of a cell expressing said gene product in the sample, thereby detecting a lung cancer cell in said sample.

6. A method for detecting a lung cancer cell, using a computer having a processor, memory, display, and input / output devices, the method comprising the steps of
a) providing a sequence of a polynucleotide isolated from a sample suspected of containing a lung cancer cell;
b) providing a database comprising
a digital computer;
a database coupled to the computer;
a database coupled to the database server having data stored therein, the data comprising records of data combined from the nucleotide obtained from the polynucleotide sequence of SEQ ID NO:16, wherein SEQ ID NO:16 is adjoined at the 5' end to a NlaIII restriction enzyme site, and a code mechanism for applying queries based upon a desired selection criteria to the data file in the database to produce reports of polynucleotide records which match the desired selection criteria; and
c) using a code mechanism for applying queries based upon a desired selection criteria to the data file in the database to produce reports of polynucleotide records of step a) which provide a match of the desired selection criteria of the sequences in the database of step b), the presence of a match being a positive indication that the polynucleotide of step a) has been isolated from a cell that is a lung cancer cell thereby identifying polynucleotides that are unique in their over-representation in lung cancer cells and not in normal lung cells, thus being particularly useful in detecting a lung cancer cell.

7. A method for screening for a potential therapeutic agent for the reversal of the neoplastic condition of a lung cancer cell wherein the cell is **characterized by** expression of a polynucleotide comprising SEQ ID NO:16 adjoined at the 5' end to an NlaIII restriction enzyme site comprising contacting a cell expressing the polynucleotide with an effective amount of a potential agent and assaying for reversal of the neoplastic condition.

8. A kit for use in a detection method according to claim 2 comprising in suitable packaging: a polynucleotide of SEQ ID NO 16, wherein SEQ ID No:16 is adjoined at the 5' end to a NlaIII restriction enzyme site or an antibody or an oligonucleotide that specifically binds to a gene product produced from a polynucleotide wherein said polynucleotide comprises a SAGE tag as shown in SEQ ID NO:16; and a reagent suitable for hybridizing or binding a sample suspected of containing the lung cancer cell.

9. A non-human transgenic animal having a disruption in a polynucleotide corresponding to a polynucleotide having a sequence selected from SEQ ID NO:16, wherein SEQ ID NO:16 is adjoined at the 5' end to a NlaIII restriction enzyme site.
